# DEMANDE DE BREVET EUROPEEN

(11) **EP 1 075 855 A1**
(43) Date de publication de la demande: **14.02.2001**
(21) Numéro de dépôt: 99420178.8
(22) Date de dépôt: 11.08.1999
(51) Int. Cl.: A61N 5/10, A61B 19/00, A61N 5/01

(54) **Dispositif de radiothérapie stéréotaxique**

(71) Demandeur: Valentin, Jean, 01220 Divonne les Bains (FR)
(72) Inventeur: Valentin, Jean, 01220 Divonne les Bains (FR)
(74) Mandataire: Savoye, Jean-Paul

(57) **Abrégé**

Ce dispositif de radiothérapie stéréotaxique par irradiation volumique d'une lésion située dans la région tête-cou, comprend une source de rayonnement appropriée au traitement, une tête radiogène (7) munie de moyens de collimation pour former un faisceau dudit rayonnement et le diriger sur la zone de traitement, des moyens de pivotement de ladite tête dont l'axe de pivotement (h) coupe l'axe (f) dudit faisceau et autour duquel ce faisceau se déplace dans un plan, un support de contention (2) de la tête du patient aptes à faire coïncider ladite lésion cérébrale avec l'intersection entre ledit axe de pivotement (h) et l'axe (f) dudit faisceau. Lesdits moyens de pivotement sont cinématiquement solidaires de moyens de guidage (4, 4', 14, 15, 14', 15') définissant une seconde trajectoire circulaire centrée autour d'un second axe (s) coupant ledit axe de pivotement (h) à son intersection avec l'axe (f) dudit faisceau et constituant l'isocentre du dispositif.

## Description

La présente invention se rapporte à un dispositif de radiothérapie stéréotaxique par irradiation volumique d'une lésion située dans la région de la tête et du cou, comprenant une source de rayonnement appropriée au traitement, une tête radiogène munie de moyens de collimation pour former un faisceau dudit rayonnement et le diriger sur la zone de traitement, des moyens de pivotement de ladite tête dont l'axe de pivotement coupe l'axe dudit faisceau et autour duquel ce faisceau se déplace dans un plan, un support de contention du corps du patient associé à des moyens d'immobilisation de sa tête et des moyens de guidage de ce support aptes à faire coïncider ladite lésion cérébrale avec l'intersection entre ledit axe de pivotement et l'axe dudit faisceau.

La présente invention trouve en particulier une application pour des traitements à l'aide d'accélérateurs médicaux d'électrons utilisés à des fins thérapeutiques humaines délivrant un faisceau, soit rayons soit un rayonnement électronique β, avec des énergies nominales comprises dans l'intervalle de 4 MeV à 50 MeV.

L'invention trouve des applications particulièrement dans le domaine de la radiothérapie stéréotaxique fractionnée et la radiochirurgie. Sans être limitatif, la présente invention s'applique notamment au traitement des tumeurs et des malformations cérébrales, que l'on désignera ci-après sous le terme de lésions, ce terme s'appliquant de façon générique à tout ce qui peut être traité par la radiothérapie ou la radiochirurgie.

Très généralement, la radiochirurgie et la radiothérapie en conditions stéréotaxiques procèdent en deux temps :
- repérage des coordonnées tridimensionnelles des lésions à traiter dans un espace de référence défini par le cadre de stéréotaxie.
- centrage et mise en oeuvre de la radiothérapie proprement dite dans le même espace de référence, matérialisé par le même cadre de stéréotaxie.

L'irradiation en conditions stéréotaxiques est utilisée pour traiter, avec une haute précision et à crâne fermé, de petits volumes intracrâniens. La radiochirurgie et la radiothérapie stéréotaxique s'adressent à des patients atteints de lésions intracrâniennes et de petites dimensions. C'est une technique de radiothérapie qui permet de traiter des tumeurs (bénignes ou malignes), des malformations artério-veineuses, des neurinomes de l'acoustique, des métastases, des méningiomes et des gliomes de petit volume, la dégénérescence maculaire liée à l'âge, la cataracte et qui remplace l'opération neurochirurgicale à crâne ouvert. L'irradiation peut être délivrée en une seule ou en plusieurs séances.

A l'heure actuelle, la balistique de l'irradiation en conditions stéréotaxiques associe soit plusieurs portes d'entrées fixes, soit une ou plusieurs irradiations en arcthérapie. Dans le cas de plusieurs portes d'entrées, l'irradiation est réalisée par de nombreuses sources radioactives de Cobalt 60 fixes, collimatées et reparties radialement sur un casque émettant individuellement des pinceaux de photons (énergie moyenne de 1.5 MeV) dont les axes sont convergents en un point focal. Dans le deuxième cas, elle est réalisée par un accélérateur linéaire d'électrons de radiothérapie conventionnelle animé d'un mouvement tournant orbital autour de la tête du patient, produisant un faisceau collimaté de photons de haute énergie (4 à 25 MeV), cylindrique et étroit dont l'axe passe toujours par un point focal, appelé isocentre où est placée la lésion à traitée. Dans les deux cas, on dispose d'une ou plusieurs sources externes de rayonnement gamma qu'il s'agit de diriger et de concentrer de façon précise sur la lésion à traiter pour faire absorber par celle-ci la dose souhaitée sans léser les tissus sains environnant la lésion. La destruction des lésions cérébrales, traitées par neurochirurgie non invasive, est obtenue par la focalisation précise du rayonnement sur la tumeur à traiter.

Le procédé d'irradiation des lésions cérébrales par utilisation des sources radioactives de Co60 est bien connu. L'appareil de traitement, a notamment été décrit dans le US-A-4780898. Il comprend un ensemble sources-collimateurs à peu près hémisphérique et une table porte-patient mobile équipée d'un dispositif destiné à coopérer avec la tête du patient. L'ensemble sources-collimateurs comporte un grand nombre de sources externes de rayons gamma associées respectivement à des canaux qui sont tous orientés vers un même point de focalisation. Alors qu'au point de focalisation la dose reçue de l'ensemble des mini-faisceaux est suffisante pour détruire la lésion, l'intensité de chaque mini-faisceau pris séparément est faible pour ne pas léser les tissus sains qu'il traverse.

Cette méthode présente des inconvénients technologiques liés à la construction mécanique des collimateurs qui sont usinés dans des blindages du genre plomb ou tungstène et sont typiquement proches en nombre de 200, leur usinage requérant des opérations longues et coûteuses. Elle présente aussi des inconvénients économiques par le besoin d'un renouvellement des sources radioactives en moyenne tous les cinq ans à cause de la durée de vie du Co60. Sur le plan médical, on dispose d'un faible nombre de dimensions de collimations disponibles dont le changement implique la remise en place précise de la tête du patient dans le système de positionnement. L'optimisation des traitements passe de manière exclusive par l'utilisation d'isocentres multiples ce qui compromet sérieusement la précision finale du traitement. Les sources étant fixes, l'utilisation des sources radioactives ne permet que l'irradiation statique du patient.

Lorsque l'irradiation stéréotaxique se réalise avec un accélérateur linéaire, l'isocentre de l'appareil de traitement coïncide avec le centre de la lésion (volume cible). Par la combinaison d'un mouvement tournant orbital de la tête radiogène autour de la tête du patient et d'un mouvement de rotation du patient, en position assise ou couchée, l'irradiation est effectuée en utilisant une série d'arcs concentriques non coplanaires (irradiation dynamique) ou des incidences multiples dans l'espace judicieusement déterminées (irradiation statique), le faisceau passe toujours par la lésion traitée tandis que les tissus qui sont situés entre source et lésion sont sans cesse renouvelés. Le champ de rayonnement des accélérateurs existants étant supérieur à 10 x 10 cm², des collimateurs additionnels de section circulaire sont adaptés pour définir de très petits faisceaux de photons de diamètre circulaire ne dépassant généralement pas les 5 cm.

Les accélérateurs linéaires de radiothérapie conventionnelle ont l'inconvénient de disposer d'un axe unique de rotation du support de la tête radiogène. Plusieurs solutions ont été proposées pour remédier à cet inconvénient. On peut les regrouper en deux classes selon la position du patient.

Selon la première catégorie, pour passer d'une arcthérapie à la suivante, le patient est traité en position assise.

Betti et al., proposent un système de positionnement et de basculement du cadre (sur deux colonnes rigides) et un fauteuil spécial, capable de tourner autour d'un axe horizontal passant par l'isocentre (chaise de traitement), pour que le corps du patient accompagne le mouvement de rotation de la tête. La cible est placée à l'isocentre de rotation du faisceau d'irradiation, par où passe aussi l'axe de rotation (transversal) du système stéréotaxique. Une combinaison de plusieurs arcs (chacun avec une position angulaire de la tête) donne un ensemble d'arcs concentriques non coplanaires.

McGinley et al. propose de fixer le bras de l'accélérateur d'électrons dans une position angulaire et de faire tourner le patient autour d'un axe vertical. Plusieurs positions angulaires du bras de l'accélérateur permettent de former un ensemble de " cônes " d'irradiations convergents. L'axe de rotation de la chaise peut être ajusté afin de compenser les éventuels défauts dus aux erreurs d'isocentrisme de la rotation du bras de l'accélérateur et de la chaise de traitement.

Avec la seconde catégorie, le patient passe d'une arcthérapie à la suivante en position couchée.

Dans ce cas, la rotation isocentrique de la table de traitement autour de l'axe vertical permet de passer d'une arcthérapie à la suivante. La tête du patient est fixée sur un support qui est lié à la table de traitement ou sur un support de tête isocentrique sur console indépendante de la table. La combinaison de la rotation isocentrique du bras de l'accélérateur linéaire avec différentes positions angulaires de la table de traitement assure l'irradiation par arc multiples. La table de traitement étant guidée à distance, un mouvement simultané et continu du bras de l'accélérateur et de la table de traitement rend possible l'irradiation dite dynamique.

Quelle que soit la position du patient, l'irradiation par un simple arc ne donnant pas les gradients d'irradiation désirés dans toutes les directions, le traitement s'effectue alors dans plusieurs positions angulaires du support de traitement (table ou chaise). La rotation du support du patient devient possible dans le cas de la combinaison accélérateur - table par un système de rotation isocentrique ou, dans le cas de la combinaison accélérateur, chaise, par un système d'inclinaison oblique du patient. L'utilisation de la combinaison accélérateur-chaise par rapport à la combinaison accélérateur-table ne présente pas d'avantage. Avec les deux systèmes, on obtient des résultats équivalents. L'efficacité de la méthode se trouve limitée par la précision du mouvement du support qui est souvent mauvaise et qui se détériore au cours du temps, alors que ce mouvement est très critique pour les irradiations stéréotaxiques.

Des améliorations ont été proposées portant sur la base et le mécanisme de rotation du support telles qu'elles sont décrites par exemple dans la demande de brevet FR N° 96 06233 déposée par Betti et al. Outre le manque de précision isocentrique des accélérateurs conventionnels, la grande distance entre la cible produisant les rayons X et le volume cible, nécessaire dans le cas des champs larges, est un facteur détériorant la qualité du traitement.

Dans les deux configurations précitées: sources radioactives, accélérateur linéaire conventionnel, le repérage de la lésion à traiter s'effectue grâce à un cadre rigide, dit cadre de stéréotaxie, positionné de manière reproductible au moyen d'implants fixés sur des points d'os judicieusement choisis sur la boîte crânienne. Ce cadre constitue par conception un référentiel orthonormé permettant de déterminer les coordonnées de n'importe quel point de la tête du patient. Selon l'emplacement et la nature de la lésion à traiter, le repérage des zones internes qui sont en cause se fait dans ce référentiel au moyen par exemple d'un scanner, d'une IRM, d'une angiographie ou toute autre méthode de localisation spatiale de ces zones. Le cadre de stéréotaxie constitue donc un repère extérieur à la tête du patient dans lequel il est possible de localiser une ou plusieurs zones cérébrales afin d'assurer leur traitement par radiothérapie.

On connaît de nombreux dispositifs permettant d'associer un référentiel à la tête d'un patient et de repérer dans ce référentiel les zones internes qui sont en cause au moyen par exemple d'une radio tomographie.

Dans l'état de la technique, le cadre de stéréotaxie sert aussi à reproduire à l'identique la position de la tête du patient pendant le traitement. Il constitue sur la tête du patient le moyen de sa mise en place précise dans le référentiel de l'appareil de traitement radiothérapeutique. Ce référentiel n'est pas le même que le référentiel des clichés, la machine de traitement étant complètement dissociée de la machine ayant servi au repérage et à la localisation spatiale de la zone à traiter. Le cadre stéréotaxique constitue ainsi le support mécanique pour la mise en place précise de la tête du patient sous l'appareil de radiothérapie.

On a aussi proposé un système de rotation du cadre stéréotaxique indépendant de la table, celle-ci servant uniquement à accompagner le mouvement du patient. La fixation au sol d'un tel système pose des problèmes et empêche l'utilisation du bras de l'accélérateur à des incidences de la partie postérieure de la tête. L'indépendance entre la fixation de la tête du patient à la colonne portant le cadre de stéréotaxie et la contention du corps du patient sur la table nécessite des systèmes de sécurité empêchant les mouvements indépendants entre les deux systèmes.

Dans ces solutions, le contrôle du positionnement de la cible, qui se fait manuellement à l'aide de vis micrométriques, est long et ne peut pas être réalisé à distance.

Le but de la présente invention est de remédier, au moins en partie, aux inconvénients des solutions susmentionnées.

A cet effet, la présente invention a pour objet un dispositif de radiothérapie stéréotaxique tel que défini par la revendication 1.

Un des avantages de cette solution est de permettre de pratiquer une irradiation d'une partie importante de la lésion sans devoir bouger le patient qui est positionné une fois, pour toute la durée du traitement.

Il est alors possible de délivrer une dose maximale à la lésion et une dose minimale aux tissus sains environnants et d'obtenir ainsi un excellent contrôle local permettant d'éviter les complications pour tout traitement radiothérapeutique s'appliquant à des cibles de faible volume. L'exactitude du volume effectivement traité par rapport au volume cible prévisionnel est aussi améliorée.

La possibilité de faire tourner la tête radiogène autour de deux axes permet d'augmenter ses positions. Le mouvement combiné autour de ces deux axes ne rend plus nécessaire la rotation isocentrique du support de traitement pour passer d'une arcthérapie à l'autre.

Avec les accélérateurs conventionnels de radiothérapie, la tête radiogène est distante des moyens de guidage en rotation de plus d'un mètre ce qui entraîne des jeux et des déformations mécaniques qui, additionnés à la dispersion du faisceau, se répercutent au niveau de la précision balistique. Grâce à la conception du dispositif stéréotaxique objet de la présente invention, susceptible de pouvoir être déplacé circulairement selon deux axes autour d'un isocentre coïncidant avec la lésion à traiter, cette distance peut être considérablement réduite. Cette diminution de la distance de la tête radiogène permet aussi de réduire la pénombre géométrique du faisceau de traitement.

Avantageusement, le dispositif de radiothérapie selon l'invention associe l'irradiation volumique dans des conditions dites stéréotaxiques, en utilisant un accélérateur linéaire d'électrons à l'imagerie, de préférence volumique en utilisant une source annexe de rayons X de l'ordre de 100 kV. Ce dispositif offrant une multiplicité d'axes de rotations et utilisant une méthode de localisation tridimensionnelle de la cible sur l'appareil de radiothérapie permet l'irradiation d'un volume cible, qui a préalablement été identifiée à l'aide des appareils et des méthodes classiques de diagnostic par imagerie.

L'association au dispositif de traitement d'un équipement d'imagerie ayant le même isocentre et bénéficiant des mêmes moyens de guidage que la tête de traitement, rend possible la localisation radiotomographique de la zone à traiter dans un système de coordonnées unique. On peut ainsi acquérir des images tomodensitométriques dans des conditions identiques à celles du traitement. Il devient alors possible de faire coïncider le centre de la zone à traiter avec l'isocentre de la machine de traitement en déplaçant la table de traitement sans ajustement mécanique préalable. En couplant le système d'imagerie avec un contrôle en ligne de la position de la zone à traiter, avant, pendant et après traitement, on obtient une bonne reproductibilité de la position du patient dans le référentiel de positionnement de la machine de traitement.

On peut alors traiter un volume cible en une ou en plusieurs séances sans qu'un cadre de stéréotaxie soit indispensable pour le positionnement du patient à chaque séance de traitement. Il ne faut, de ce fait, plus prépositionner le système de contention au moyen d'une simulation de la cible par un fantôme, ce qui allège substantiellement la procédure de traitement. L'absence du cadre stéréotaxique lors du traitement présente l'avantage d'éliminer des éléments encombrants autour de la tête du patient lors de l'étape de traitement, ce qui permet une grande souplesse lors du choix des angles d'incidences des faisceaux. Elle réduit aussi les risques de collision avec la tête radiogène.

La présente invention sera mieux comprise à la lecture de la description suivante d'une forme d'exécution du dispositif de radiothérapie stéréotaxique objet de cette invention, donnée à titre d'exemple et illustrée très schématiquement à l'aide des dessins annexés.
La figure 1 est une vue en élévation latérale de cette forme d'exécution;
la figure 2 est une vue en élévation selon II-II de la figure 1;
la figure 3 est une vue de dessus de la figure 1;
la figure 4 est une vue semblable à la figure 2 dans une autre position de la tête radiogène;
la figure 5 est une vue en élévation latérale lorsque le dispositif est utilisé avec le système d'imagerie;
la figure 6 est une vue de face de la tête d'un patient sur laquelle est indiqué l'angle de déplacement coronal de la tête radiogène;
la figure 7 est une vue latérale de la tête d'un patient avec l'angle de déplacement dans le plan sagittal de la tête radiogène;
la figure 8 est une vue agrandie, en coupe selon VIII-VIII de la figure 2;
la figure 9 est une vue selon IX-IX de la figure 9;
la figure 10 est une vue partielle, agrandie en perspective de l'extrémité de la table de contention portant les moyens de positionnement du cadre stéréotaxique.

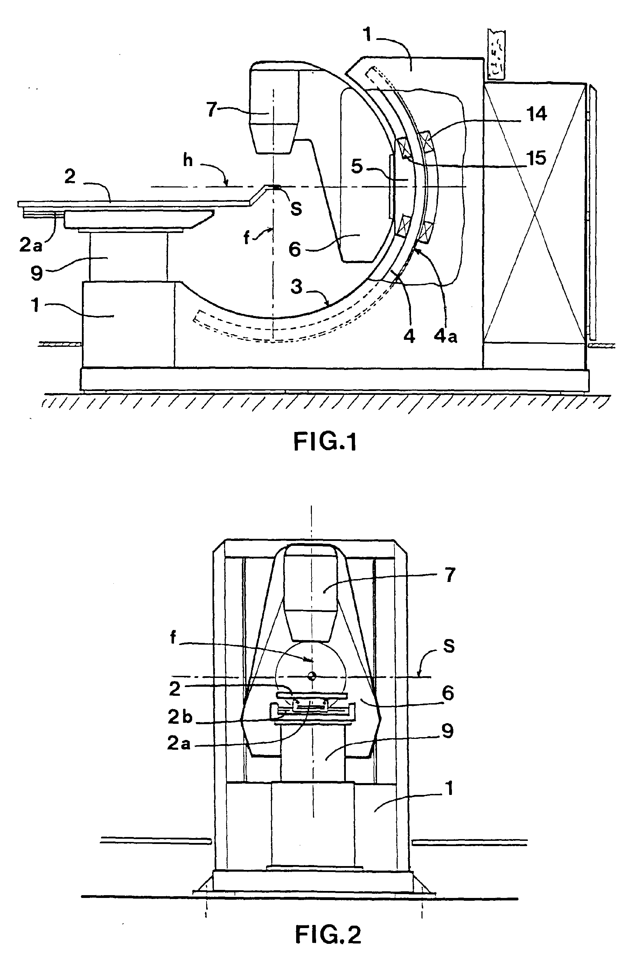

Le dispositif illustré par les figures 1-4 comporte un bâti 1 comprenant, de manière solidaire, un support pour recevoir le corps du patient constitué par une table 2. Le dessous de cette table 2 porte deux jeux de coulisses horizontales 2a, 2b dont les axes de déplacement se coupent à angle droit, l'axe de la coulisse 2a étant longitudinal et celui de la coulisse 2b étant transversal. Ces coulisses, 2a, 2b permettent des déplacements indépendants, de précision micrométrique. Le tout est supporté par une colonne 9 montée coulissante dans le bâti 1 selon un axe vertical et susceptible de se déplacer avec une précision micrométrique. Les déplacements de la table 2 selon ces trois axes peuvent avantageusement être commandés à distances par des moyens non représentés dans la mesure où ils ne font pas partie de la présente invention.

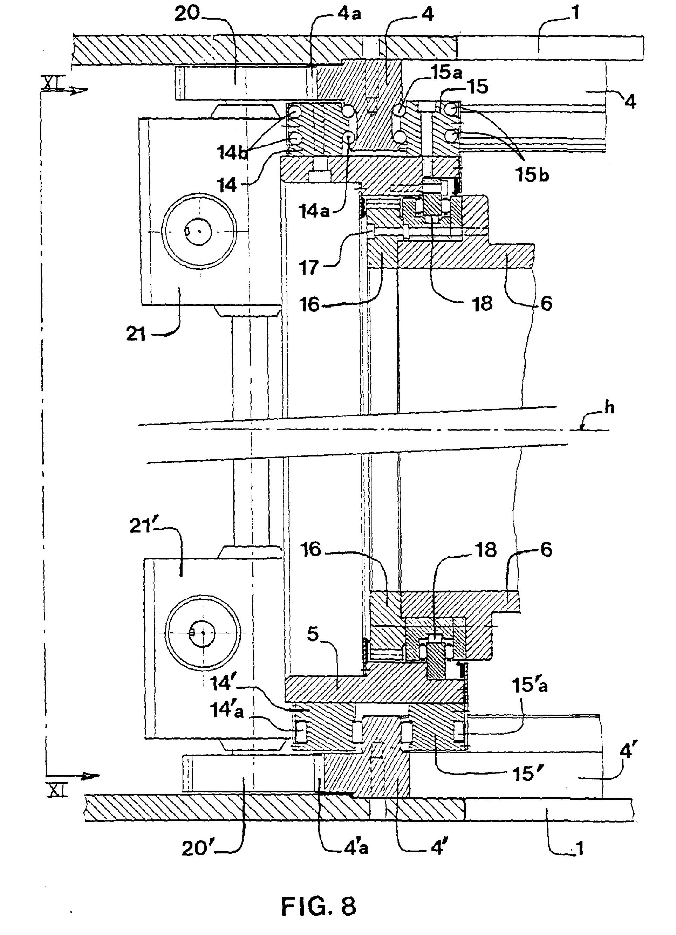

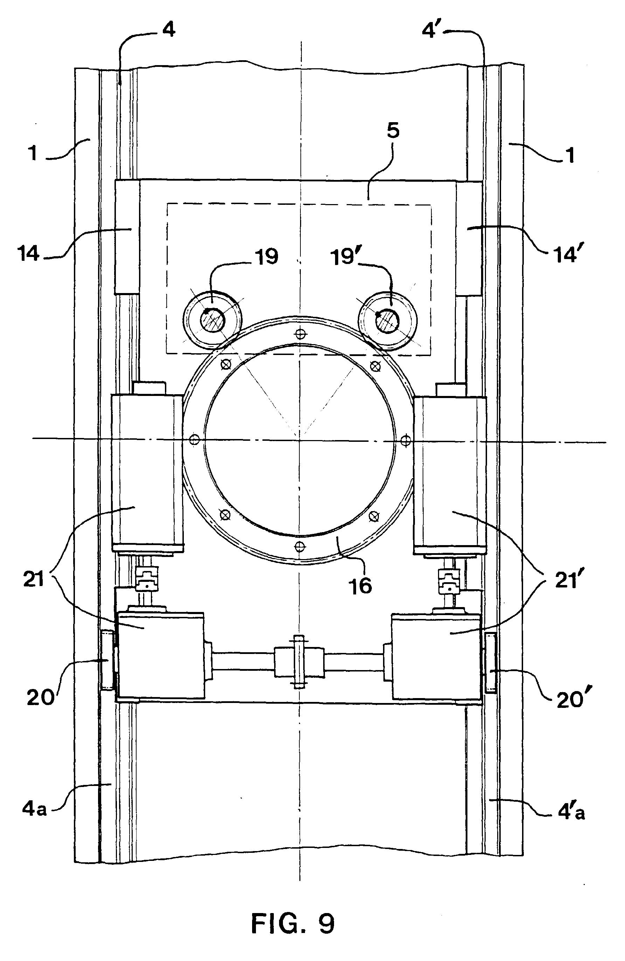

Le bâti 1 comporte encore une ouverture semi-circulaire 3 formée autour d'un axe transversal contenu dans le plan horizontal. Deux rails de guidage curviligne parallèles 4, 4' sont fixé au bâti 1 coaxialement à l'ouverture 3. Chacun de ces rails (figures 8 et 9) présente une crémaillère 4a sur sa face convexe. Un chariot 5 est muni d'organes de roulement 14, 14', 15, 15' en prise avec les rails de guidage 4, 4' et de deux pignons moteurs 20, 20', en prise avec les crémaillères respectives 4a, 4'a. Grâce à ces rails de guidage curviligne 4, 4' le chariot 5 peut se déplacer dans le plan sagittal, selon une trajectoire circulaire centrée sur un axe s. Ce chariot 5 porte un bras 6 de support d'une tête radiogène 7 et est monté pivotant autour d'un axe h qui coupe l'axe s. Le point d'intersection des axes h et s est l'isocentre du dispositif de traitement stéréotaxique. Il résulte de cette disposition que le bras 6 peut se déplacer autour des axes h et s. Le déplacement du bras 6 autour des axes h et s permet de balayer ainsi une surface sphérique centrée sur l'isocentre du dispositif de radiothérapie.

La figure 8 illustre plus en détail le système de guidage et d'entraînement du chariot 5 sur les rails de guidage circulaires 4, 4'. Le chariot 5 porte des paires de patins 14, 15 arqués selon un même rayon que les rails de guidage 4. Ces patins 14, 15 situés d'un côté du chariot 5 sont du type connu dit à recirculation de billes 14a, 15a. Les patins 14 et 15 présentent chacun deux pistes sans fin de circulation parallèles 14b, respectivement 15b pour les billes 14a, respectivement 15a. Plusieurs de ces paires de patins peuvent être réparties le long des rails de guidage 4, 4'. Les patins 14', 15' situés de l'autre côté du chariot 5 sont du même type mais les billes 14a, 15a sont remplacées par des rouleaux 14'a, 15'a, afin que le positionnement axial du chariot 5 soit déterminé par les patins 14 et 15 et les billes 14a, 15a.

On peut encore voir sur cette figure 8 le système de pivotement et d'entraînement du bras 6 de support de la tête radiogène 7. Ce bras 6 est monté pivotant sur le chariot 5 par un paliers annulaire à roulement 18 du type SKF YRT460. Ces systèmes de guidage et de pivotement permettent d'éviter les jeux et donc de garantir une grande précision dans le déplacement de la tête radiogène. Une couronne d'entraînement 16 est fixée au bras 6 par des vis 17. On voit en particulier sur la figure 9 que cette couronne d'entraînement est en prise avec des pignons d'entraînement 19 solidaires d'un moto-réducteur (non représenté).

Cette figure 9 permet encore de voir les pignons d'entraînement 20, 20', en prise avec les crémaillères 4a, 4'a, entraînés par deux moto-réducteurs 21 et 21'.

La tête radiogène 7 comprend deux types de moyens pour collimater alternativement les rayonnements émis sélectivement par deux sources de rayonnements, l'une constituée par un accélérateur linéaire destiné à produire des rayons X, l'autre par une source de rayon X de bas voltage à faible dose. Les détails de cette tête radiogène et de ces sources ne faisant pas partie de la présente invention, ils ne seront pas représentés ni décrits dans la mesure où ils ne sont pas nécessaires à la compréhension de cette invention. Les deux faisceaux de rayonnement collimatés sortant de la tête radiogène 7 ont un axe f commun qui passe par l'intersection des axes de pivotement h et s de cette tête radiogène, correspondant à l'isocentre du dispositif. En tournant autour de l'axe h, l'axe f du faisceau se déplace dans un plan.

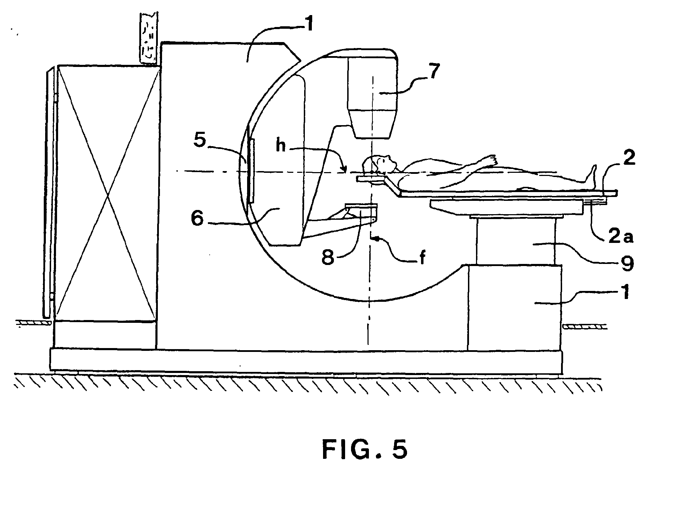

Comme illustré par la figure 5, le bras 6 porte encore, de préférence, une surface réceptrice escamotable servant de détecteur mono ou bi-dimensionnels 8 sensible aux rayons X utilisés dans cet exemple, pour réaliser une image de la lésion à traiter. Ce détecteur 8 est disposé sur la trajectoire du faisceau formé par la tête radiogène 7, au-delà de d'isocentre situé à l'intersection des axes h, s et f, de sorte qu'il détecte les rayons X après que le faisceau collimaté a traversé la tête du patient. Les moyens pour produire sur le même axe f deux faisceaux de rayons X, l'un servant pour l'imagerie de diagnostic, l'autre pour le traitement, sont décrits par exemple dans le US 5 471 516 et sont susceptibles d'être utilisés pour la tête radiogène 7 de la présente invention.

Grâce à cette possibilité de collimater deux faisceaux d'énergies différentes sur un même axe, le dispositif selon la présente invention travaille toujours dans le même système de coordonnées par rapport au même isocentre rendant ainsi possible de procéder successivement à la localisation radiotomographique de la zone à traiter puis au traitement de la lésion ainsi localisée, au moyen de rayons gamma.

Le détecteur 8 délivre les données sous forme numérique qui sont enregistrées au moyen d'un système informatique et sont reconstruites en trois dimensions à l'aide d'un logiciel spécifique de façon connue dans les systèmes d'imagerie médicale. L'image reconstruite est destinée à observer soigneusement la région de la tête du patient que l'on traite, en fournissant une image de haute définition et de haut contraste de cette région. Selon la figure 5, cette image permet de déplacer à distance la table de contention 2 pour amener le centre de la lésion à traiter à l'isocentre du dispositif de traitement.

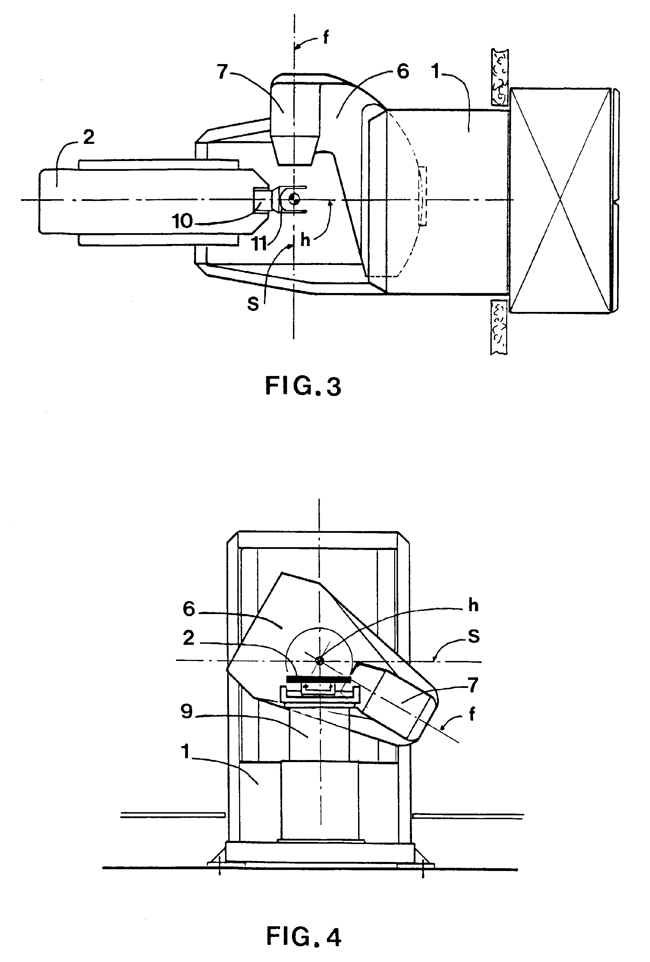

La figure 3 est une vue de dessus de ce dispositif de radiothérapie dont la tête radiogène 7 a été déplacée autour de l'axe h dans le plan orbital et correspond à la position illustrée par la figure 4. A son extrémité voisine de l'isocentre, la table 2 peut porter un cadre stéréotaxique 11 interchangeable. Bien que ce cadre de stéréotaxie 11 ne soit plus indispensable pour positionner et traiter la tête du patient, il reste utile pour évaluer des erreurs au moyen de simulations par fantôme, pour mesurer périodiquement la stabilité de l'isocentre de la machine ou établir des procédures d'alignement des faisceaux.

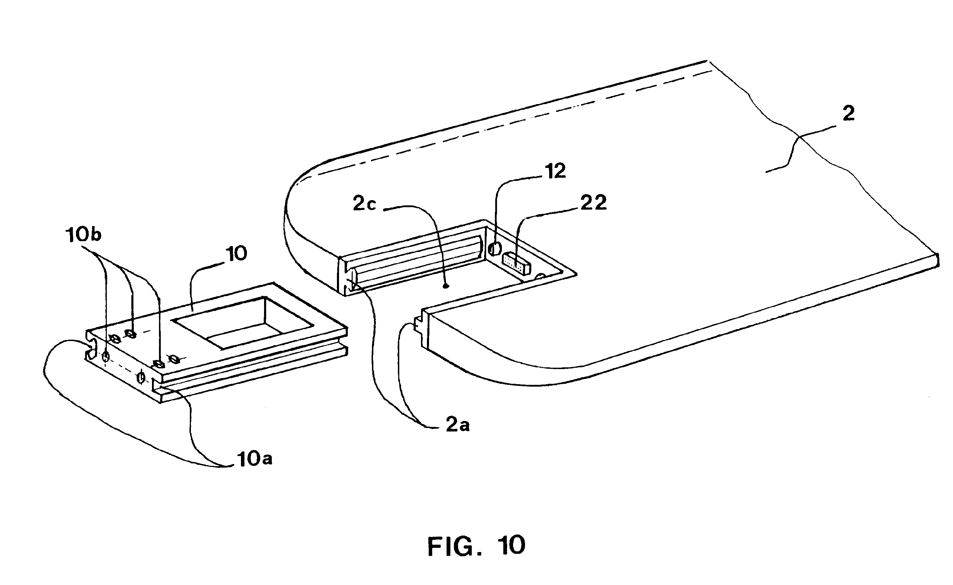

La table 2 comprend à cet effet un support et des adaptateurs universels 10, dont est représenté sur les figures 3 et 10, pour le positionnement des différents cadres de stéréotaxie 11 existants; ce qui rend possible la fixation à tout instant d'un cadre stéréotaxique 11 pour effectuer un repérage dans un système de coordonnées externes et pouvoir réaliser des simulations. Chaque cadre de stéréotaxie 11 peut être muni d'un adaptateur universel 10 spécifique laissé à demeure, ce qui permet un repositionnement rapide.

La figure 10 montre plus en détail les moyens de positionnement de l'adaptateur universel 10 sur la table 2. Cette table 2 présente à une extrémité une ouverture rectangulaire 2c comportant deux glissières 2d ménagées sur deux côtés opposés de cette ouverture 2c. Ces glissières 2d sont destinées à venir respectivement en prise avec deux rainures 10a ménagées le long de deux bords opposés de l'adaptateur 10. Le fond de l'ouverture rectangulaire 2c porte deux butées 12, dont seule l'une est visible, à l'intérieur desquelles se trouve un commutateur de précision. Ce commutateur est par exemple un commutateur de Baumerlectric, de type My-Com, qui permet de déterminer une position avec une précision de 1 µm.

Entre les deux butées 12 se trouve une connexion 22 destinée à un système de codage électronique sur 8 bits destiné à permettre de transmettre ses données afin de déterminer si le cadre de stéréotaxie mis en place correspond bien au patient devant subir le traitement. Grâce à ce positionnement de grande précision et à ce système d'identification, il est possible de traiter un même patient en plusieurs séances et en retrouvant chaque fois rapidement et avec sécurité la position de référence par rapport à l'isocentre du dispositif.

Lorsque l'adaptateur universel 10 est positionné avec précision dans l'ouverture 2c de la table 2, on procède à l'étalonnage de sa face supérieure et de sa face frontale avant, en mesurant les distances respectives de ces faces avec l'isocentre du dispositif. Cet adaptateur universel comporte, sur ses faces supérieure, respectivement frontale avant, une série d'alésages de précision lOb destinés au positionnement précis des différents types de cadres de stéréotaxie.

Tout autre moyen d'immobilisation approprié de la tête du patient peut remplacer avantageusement le cadre de stéréotaxie. Cette immobilisation peut ainsi être obtenue, par exemple, à l'aide de masques thermoformables. En effet, le cadre de stéréotaxie habituel constitue souvent une gène pour le déplacement de la tête radiogène 7.

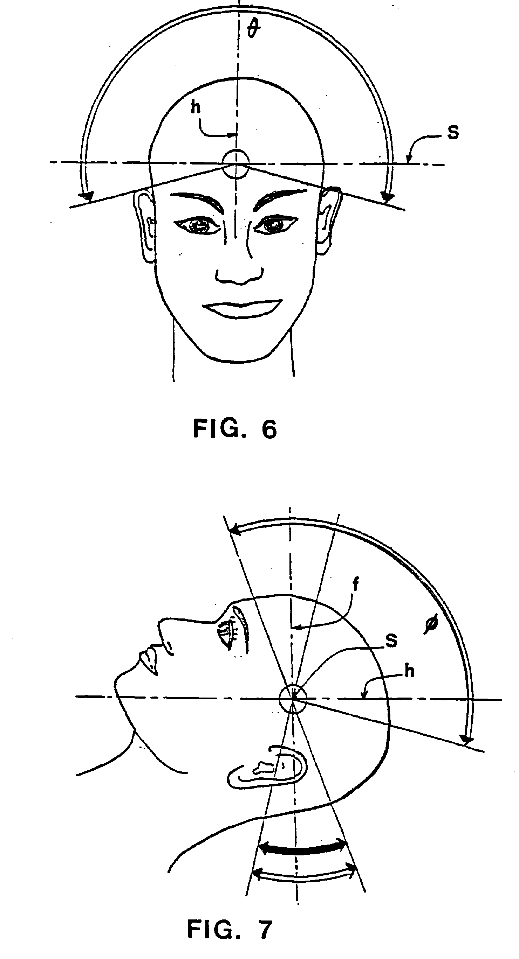

Les figures 6 et 7 illustrent respectivement et à titre d'exemple, les angles θ, respectivement φ correspondant aux angles susceptibles d'être parcourus par la tête radiogène 7 par rapport à l'isocentre dans les plans orbital, respectivement sagittal autour de la tête du patient. Ces déplacements angulaires peuvent être combinés simultanément ou alternativement pour effectuer une irradiation du volume de la lésion à traiter centrée sur l'isocentre du dispositif de radiothérapie en minimisant les effets sur les tissus sains adjacents. Cette capacité de déplacement angulaire permet d'étendre le traitement à des zones jusqu'à présent impossibles d'accès.

## Revendications

1. Dispositif de radiothérapie stéréotaxique par irradiation volumique d'une lésion située dans la région tête-cou, comprenant une source de rayonnement appropriée au traitement, une tête radiogène (7) munie de moyens de collimation pour former un faisceau dudit rayonnement et le diriger sur la zone de traitement, des moyens de pivotement de ladite tête dont l'axe de pivotement (h) coupe l'axe (f) dudit faisceau et autour duquel ce faisceau se déplace dans un plan, un support de contention (2) du corps du patient associé à des moyens d'immobilisation de sa tête et des moyens de guidage (2a, 9, 10) de ce support de contention (2) aptes à faire coïncider ladite lésion cérébrale avec l'intersection entre ledit axe de pivotement (h) et l'axe (f) dudit faisceau, caractérisé en ce que lesdits moyens de pivotement sont cinématiquement solidaires de moyens de guidage (4, 4', 14, 15, 14', 15') définissant une seconde trajectoire circulaire centrée autour d'un second axe (s) coupant ledit axe de pivotement (h) à son intersection avec l'axe (f) dudit faisceau et constituant l'isocentre du dispositif.

2. Dispositif selon la revendication 1, caractérisé en ce que des moyens d'imagerie médicale sont associés auxdits moyens de pivotement, pour déterminer la position stéréotaxique de la lésion à traiter par rapport audit isocentre dans un même système de coordonnées.

3. Dispositif selon la revendication 2, caractérisé en ce que, pour former lesdits moyens d'imagerie, une source de rayons X est reliée à ladite tête radiogène (7) et que des moyens de détection (8) mono ou bi-dimensionnels sont disposés sur la trajectoire d'un faisceau de rayons X collimaté par ladite tête radiogène (7), cette dernière et lesdits moyens de détection (8) étant situés de part et d'autre dudit isocentre.

4. Dispositif selon l'une des revendications précédentes, caractérisé en ce que lesdits moyens de guidage (4, 4', 14, 15, 14', 15') de ladite tête radiogène (7) et dudit support de contention (2) sont solidaires d'un même bâti (1) .

5. Dispositif selon l'une des revendication précédentes, caractérisé en ce que ledit axe de pivotement (h) et ledit second axe (s) autour duquel est centrée ladite seconde trajectoire circulaire se coupent à angle droit.

6. Dispositif selon l'une des revendications précédentes, caractérisé en ce que lesdits moyens de guidage cinématiquement solidaires desdits moyens de pivotement de ladite tête radiogène (7) comportent un rail curviligne (4, 4') centré sur ledit second axe (s), des organes de roulement (14, 14', 15, 15') solidaire de ladite tête radiogène (7) en prise avec ledit rail curviligne (4, 4'), une crémaillère (4a, 4'a) solidaire de ce rail curviligne (4, 4') et des pignons (20, 20') solidaire de ladite tête radiogène (7), en prise avec ladite crémaillère (4a, 4'a) pour contrôler le déplacement de cette tête par rapport audit rail curviligne.

7. Dispositif selon l'une des revendications précédentes, caractérisé en ce qu'il comporte un organe d'adaptation universel (10) de cadres de stéréotaxie (13) audit support de contention (2), des moyens (2d, 10a, 12) pour déterminer la position d'éléments de référence solidaires dudit organe d'adaptation (10) par rapport à l'isocentre du dispositif et des moyens de positionnement universel (lOb) desdits cadres de stéréotaxie (13).

8. Dispositif selon la revendication 6, caractérisé en ce que ledit organe d'adaptation universel (10) comporte des moyens de codage électroniques et des moyens de connexion pour coopérer avec des moyens de connexion (12) et de codage (22) solidaires dudit support de contention (2) et permettre la lecture à distance desdits moyens de codage.
